# FASCICULE DE BREVET EUROPEEN

(11) **EP 4 543 288 B1**
(45) Date de publication et mention de la délivrance du brevet: **10.06.2026**
(21) Numéro de dépôt: 23738570.3
(22) Date de dépôt: 21.06.2023
(51) Int. Cl.: A61B 5/103, A61B 5/00

(54) **SYSTÈME DE PLATEFORME DE FORCE UTILISABLE DANS UN ARTICLE CHAUSSANT POUR LA MESURE DU POIDS ET DE LA PRESSION EXERCÉS PAR LE PIED D'UN PATIENT**
IN EINEM SCHUHWERK VERWENDBARES KRAFTPLATTFORMSYSTEM ZUR MESSUNG DES GEWICHTS UND DES DRUCKS, DER VOM FUSS EINES PATIENTEN AUSGEÜBT WIRD
FORCE PLATFORM SYSTEM USEABLE IN AN ITEM OF FOOTWEAR TO MEASURE THE WEIGHT AND THE PRESSURE EXERTED BY THE FOOT OF A PATIENT

(30) Priorité: 22.06.2022 FR 2206142
(43) Date de publication de la demande: 30.04.2025
(73) Titulaire: Fluid Innov, 42500 Le Chambon-Feugerolles (FR)
(72) Inventeur: EMPEREUR, Julien, 73320 TIGNES (FR)
(74) Mandataire: Germain Maureau
(86) Numéro de dépôt international: PCT/FR2023/050931
(87) Numéro de publication internationale: WO 2023/247897

(56) Documents cités:
- US-A- 6 122 846
- US-A1- 2021 046 356

## Description

### Domaine technique

La présente invention concerne généralement le domaine des systèmes baropodométrie utilisés notamment pour la rééducation des patients ayant subi une blessure à un membre inférieur, ou présentant une déficience neurologique de sensibilité des membres inférieurs. La présente invention concerne en particulier un système de mesure du poids appliqué par un patient sur son pied.

### Etat de la technique

Le document US 6 122 846 décrit un dispositif de chaussure pour surveiller les forces appliquées au pied d'un utilisateur, en utilisant un capteur de déformation pour mesurer les forces transmises via une structure de support et fournir des indications si elles dépassent des limites prédéfinies. Le document US 2021/0046356 divulgue un système d'analyse de la marche pour la détection précoce des troubles du pied chez les patients diabétiques, en mesurant la température ainsi que les forces verticales et de cisaillement à l'aide de capteurs intégrés dans des semelles intérieures.

Le brevet FR 3 072 016 déposé par le Demandeur décrit un système de mesure du poids exercé par le pied d'un patient durant la marche ou en station debout. Ce système comprend un article chaussant adapté à la rééducation des jambes. Ce système est pourvu d'une semelle dans laquelle sont intégrés des capteurs de pression ou de force reliés à une unité de traitement. L'unité de traitement est configurée pour traiter en temps réel les signaux issus des capteurs et fournir en temps réel au patient ou au rééducateur des indications sur le poids exercé par le pied du patient sur la semelle.

Cependant, le mode d'intégration des capteurs dans la semelle et le mode de fixation de la semelle à l'article chaussant ne permet pas de mesurer le poids total exercé par le pied du patient sur la semelle. En effet, une partie du poids du patient est transmise directement de la semelle à l'article chaussant et au sol sans pouvoir être mesuré par les capteurs. Les indications susceptibles d'être fournies en temps réel par les capteurs sont donc erronées
Il est donc souhaitable de proposer un article chaussant adapté à la rééducation des jambes, permettant de mesurer la force totale exercée par le pied d'un patient sur une semelle. Il est également souhaitable de pouvoir indiquer en temps réel au patient et au rééducateur une information aussi précise que possible concernant les forces d'appui exercées par le pied du patient et leur répartition. Il peut également être souhaitable de recueillir une information relative à la répartition des forces exercées par le pied du patient.

### Résumé

Des modes de réalisation concernent un dispositif de mesure d'une force d'appui exercée par le pied d'un patient, le dispositif comprenant : un support, une semelle intérieure prévue pour supporter le pied du patient, maintenue sur le support, le support et la semelle étant agencés pour que, lorsque le pied du patient est en appui sur la semelle intérieure, les forces exercées par le pied du patient sur la semelle intérieure soient transmises au support en un ensemble fini de zones de contact séparées ; des capteurs interposés entre le support et la semelle intérieure respectivement aux zones de contact, et configurés chacun pour fournir un signal représentatif d'une force exercée sur la zone de contact correspondante ; et une unité de traitement connectée aux capteurs et configurée pour acquérir les signaux de force fournis par les capteurs.

Grâce à ces dispositions, l'ensemble des forces exercées par le pied du patient sur la semelle peut être mesuré, ce qui permet une meilleure précision pour guider le patient dans ses exercices de rééducation ou de stimulation neuromusculaire.

Selon un mode de réalisation, le dispositif comprend un organe de retenue agencé entre la semelle intérieure et le support en une zone de retenue distincte des zones de contact, pour solidariser la semelle intérieure au support sans transmettre les forces exercées sur la semelle au support.

Selon un mode de réalisation, l'organe de retenue est configuré pour s'engager partiellement dans un orifice au travers du support et venir se fixer dans la semelle intérieure, de sorte que l'organe de retenue soit retenu par le support vers le haut avec un jeu non nul.

Ainsi, la semelle est retenue dans la coque sans interférer avec les mesures de force.

Selon un mode de réalisation, le support comprend une semelle extérieure de forme bombée le long d'un axe longitudinal du support.

En effet, une telle forme bombée améliore l'entrainement et accélère la rééducation des patients.

Selon un mode de réalisation, les capteurs comprennent trois capteurs de force incluant un capteur disposé sous une partie arrière de la semelle intérieure, et deux capteurs disposés sous une partie avant de la semelle intérieure.

Cette disposition des capteurs permet de localiser précisément le centre de pression exercé par le pied sur la semelle.

Selon un mode de réalisation, le support présente la forme d'une coque avec un rebord entourant la semelle.

Selon un mode de réalisation : l'unité de traitement est configurée pour calculer en temps réel une valeur de force appliquée par le pied du patient sur la semelle intérieure à partir des signaux fournis par les capteurs, et transmettre la valeur de force vers un équipement externe, et/ou l'unité de traitement est configurée pour transmettre en temps réel des mesures représentatives des signaux fournis par les capteurs à l'équipement externe, l'équipement externe étant configuré pour calculer en temps réel une valeur de force appliquée par le pied du patient sur la semelle intérieure à partir des mesures fournies par l'unité de traitement.

Le calcul en temps réel de la force exercée offre la possibilité de réaliser une boucle de rétroaction permettant au patient d'ajuster en temps réel la force qu'il exerce sur la semelle.

Des modes de réalisation peuvent également concerner un système de rééducation d'un membre inférieur d'un patient, le système comprenant : un dispositif de mesure tel que précédemment défini, et une interface homme-machine en communication avec l'unité de traitement du dispositif de mesure et configurée pour émettre en temps réel un signal représentatif d'une valeur de force calculée à partir des signaux de force fournis par les capteurs.

La présence d'une interface homme/machine fournissant en temps réel des signaux représentatifs de la force exercée sur la semelle permet de réaliser cette boucle de rétroaction.

Selon un mode de réalisation, l'interface homme-machine comprend un terminal mobile en communication avec l'unité de traitement et muni d'une application dédiée configurée pour traiter des signaux transmis par l'unité de traitement.

Grâce à l'utilisation d'un terminal mobile par exemple de type "smartphone" la réalisation d'une interface homme-machine complexe peut être réalisée facilement sous la forme d'une application dédiée installée dans le terminal.

Selon un mode de réalisation, l'interface homme-machine est configurée pour émettre en temps réel : un premier signal lumineux et/ou sonore lorsque la valeur de force est comprise dans une première plage de valeurs de forces, et un second signal lumineux et/ou sonore lorsque la valeur de force est comprise dans une seconde plage de valeurs de forces, supérieure à la première plage de valeurs de force, et inférieure à une valeur de consigne.

La production de tels stimuli simplifiés permet au patient d'utiliser correctement le système presque instantanément.

Selon un mode de réalisation, l'unité de traitement ou l'interface homme-machine est configurée pour calculer en temps réel la position d'un centre de pression situé au barycentre des forces exercées sur les zones de contact et mesurées par les capteurs.

Le calcul en temps réel de la position du centre de pression permet de suivre l'évolution de la posture du patient.

Selon un mode de réalisation, l'interface homme-machine est configurée pour afficher la position du centre de pression en relation avec le contour d'un pied humain, et/ou des variations au cours du temps de la position du centre de pression sous forme de chronogrammes et/ou en relation avec le contour d'un pied humain.

L'affichage et la mise à jour en temps réel de la position du centre de pression permet au patient d'effectuer des corrections de posture, et au thérapeute de guider le patient dans ces corrections.

### Brève description des figures

La présente invention sera bien comprise à l'aide de la description qui suit en référence aux figures annexées, dans lesquelles des signes de références identiques correspondent à des éléments structurellement et/ou fonctionnellement identiques ou similaires.
La figure 1 représente schématiquement, en perspective de trois quart arrière, un dispositif de mesure de forces exercées par le pied d'un patient, selon un mode de réalisation,
La figure 2 représente schématiquement en perspective de trois quart avant, du dispositif de mesure sans une semelle intérieure, selon un mode de réalisation,
La figure 3 est une vue schématique en perspective éclatée du dispositif, selon un mode de réalisation,
La figure 4 représente schématiquement le dispositif, en coupe suivant un plan longitudinal médian vertical, selon un mode de réalisation,
La figure 5 représente schématiquement un patient équipé du dispositif de mesure,
La figure 6 représente schématiquement des circuits d'un système de mesure et d'entrainement, selon un mode de réalisation,
Les figures 7A, 7B, 7C et 7D représentent schématiquement un terminal mobile en communication avec le système de mesure et exécutant une application dédiée,
Les figures 8A, 8B représentent des chronogrammes de variation de la position d'un centre de pression du pied respectivement vers l'avant ou l'arrière du pied, et vers la gauche ou la droite du pied, pendant le déroulé du pas,
Les figures 9A, 9B représentent des exemples de courbes de variation de la position du centre de pression du pied en statique, affichées en surimpression sur une image du contour d'un pied d'un utilisateur.

### Description détaillée

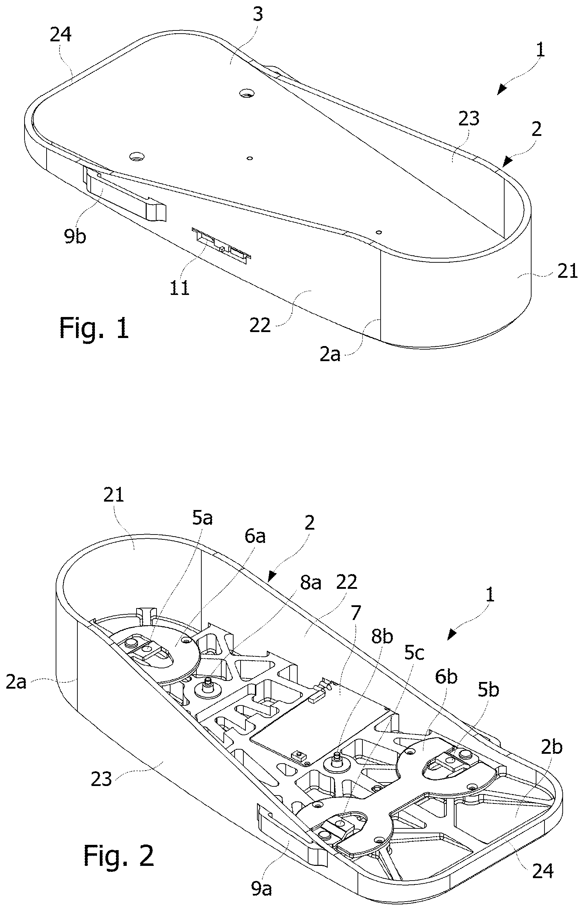

Les figures 1 à 4 représentent un dispositif de mesure de forces exercées par le pied d'un patient, notamment en station debout et/ou durant la marche. Le dispositif de mesure se présente sous la forme d'un chaussant 1 comprenant une coque 2 supportant une semelle intérieure 3 et comportant une semelle extérieure 10. La coque 2 comprend un fond 2b et un rebord 2a entourant la semelle intérieure 3. Le rebord 2a comporte une partie arrière 21 (côté talon) et des parties latérales 22, 23 s'étendant de la partie arrière 2a vers l'avant du chaussant 1, et une partie avant 24 (côté avant du pied). La hauteur du rebord 2a diminue de l'arrière vers l'avant du chaussant 1 où le rebord ne n'excède pas l'épaisseur de la semelle 3. Le chaussant 1 représenté sur les figures 1 à 4 est prévu par exemple pour un pied gauche, ou indifféremment pour un pied gauche et un pied droit. La semelle intérieure 3 est retenue dans la coque 2 par le rebord 2a formé par les parties 21, 22, 23 et 24. La coque 2 peut être fabriquée par moulage d'une matière plastique.

La coque 2 présente des logements prévus pour recevoir des supports 6a, 6b de capteurs. Le support 6a est disposé dans un logement dans la région arrière (région du talon) du chaussant 1 et supporte un capteur 5a. Le support 6b est disposé dans une région avant du chaussant 1 et supporte deux capteurs 5b, 5c, de sorte que le capteur 5b est disposé dans une région avant gauche et le capteur 5c dans une région avant droite du chaussant. La semelle intérieure 3 repose exclusivement sur les capteurs 5a, 5b, 5c, de sorte que l'intégralité des forces appliquées sur le dessus de la semelle 3 soit transmise aux capteurs. Ainsi, les capteurs 5a, 5b, 5c sont interposés entre la coque 2 et la semelle 3 aux seules zones de contact entre ces dernières, y compris lorsque le patient exerce tout son poids sur la semelle 3. La présence d'au moins trois capteurs de force répartis dans la coque 2 permet d'éviter un basculement de la semelle 3 et donc que la semelle prenne appui sur la coque en d'autres zones d'appui que celles où sont placés les capteurs 5a, 5b, 5c.

La coque 2 présente également un logement prévu pour recevoir une carte de circuit électronique 7, et un logement prévu pour recevoir une batterie d'alimentation électrique de la carte 7. Le logement de la batterie 7 peut par exemple être accessible par une fente latérale 11 formée dans la partie gauche 22 du rebord 2a de la coque 2. La carte de circuit électronique 7 est connectée aux capteurs 5a, 5b, 5c.

Les parties latérales 22, 23 du rebord de la coque 2 peuvent comporter des passants 9a, 9b solidaires de l'extérieur de la coque, pour le passage de sangles de maintien du chaussant 1 sur le pied, à la manière d'une sandale. Sur la f igure 4, le chaussant 1 peut également comprendre des languettes 12 s'étendant vers le haut depuis les parties latérales 22, 22 du rebord 2a de la coque 2 pour permettre de fixer le chaussant 1 autour de la cheville du patient.

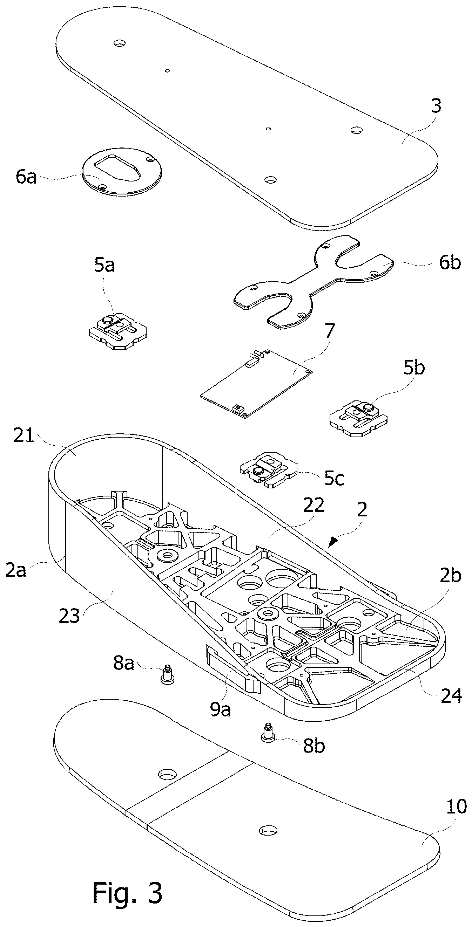

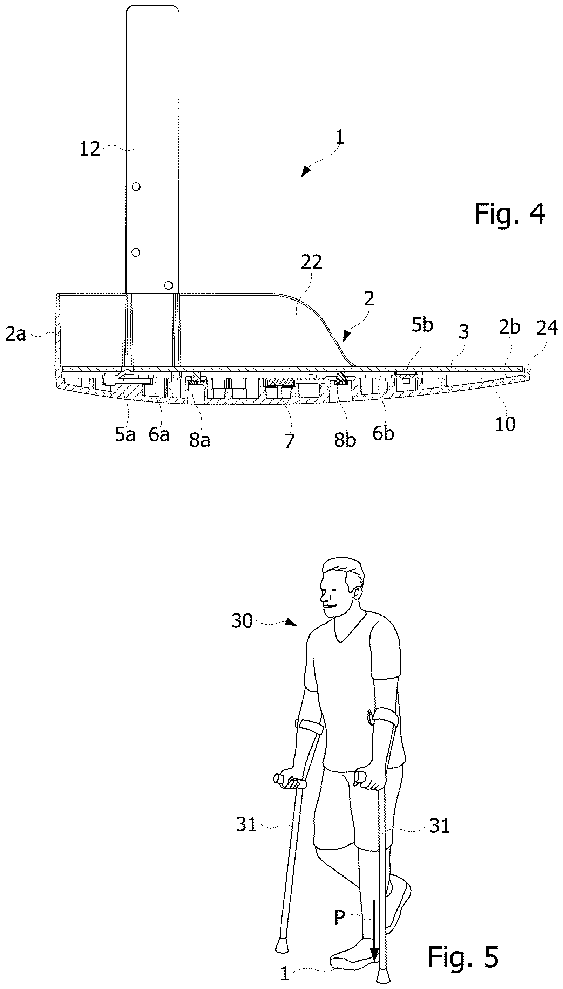

Sur les figures 3 et 4, la semelle intérieure 3 peut être maintenue dans la coque 2 par au moins une vis 8a, 8b. A cet effet, chaque vis 8a, 8b est introduite dans un orifice au travers de la coque 2 pour venir se visser dans la semelle intérieure 3. La tête de chaque vis 8a, 8b est logée dans une cavité formée dans la coque 2 et débouchant dans la semelle extérieure 10, de sorte que la tête de la vis ne vient pas en appui sur la coque 2 ou sur la semelle extérieure 10 lorsqu'une pression est exercée sur la semelle intérieure 3. De cette manière, chaque vis 8a, 8b permet seulement de retenir la semelle intérieure 3 à l'intérieur de la coque 2 et ainsi de l'empêcher de tomber hors de la coque. En revanche, les vis 8a, 8b ne transmettent aucune force exercée sur la semelle intérieure 3 vers la coque. Dans l'exemple des figures, deux vis 8a, 8b sont utilisées pour maintenir la semelle intérieure 3, sachant qu'une seule vis peut être suffisante et que d'autres vis agencées de cette manière peuvent être ajoutées. Il est à noter qu'il est préférable de laisser un certain jeu entre les têtes des vis 8a, 8b et la coque 2, afin de prévenir tout risque de transmission de force entre la semelle et la coque par les vis 8a, 8b. Ainsi, la semelle (3) peut être maintenue dans la coque avec un léger jeu vers le haut.

Sur la figure 4, la semelle extérieure 10 est légèrement bombée suivant un axe longitudinal du pied du patient. En effet, il s'avère qu'une telle forme bombée améliore l'entrainement et accélère la rééducation des patients.

Les capteurs 5a, 5b, 5c peuvent être de type capteur de force pour mesurer chacun en temps réel une valeur de force exercée par une zone respective du pied du patient sur la semelle intérieure 3. Par exemple, les capteurs de force 5a, 5b, 5c sont de type jauge de contrainte ou de pression, et sont sollicitées en compression, selon une direction perpendiculaire à la semelle intérieure 3. Les jauges de contrainte peuvent être montées dans des ponts de Wheatstone pour une plus grande sensibilité. Les jauges de contrainte présentent de nombreux avantages et notamment, elles se déforment peu et donc résistent mieux dans la durée. Elles offrent également une grande précision d'étalonnage et de mesure, et présentent faible encombrement compatible avec celui disponible dans la coque, et un coût réduit. D'autres types de capteurs de force peuvent être employés sans sortir du cadre de la présente description.

La figure 5 représente un patient 30 équipé de béquilles 31, par exemple en raison d'une blessure d'un ou des membres inférieurs. Le patient 30 a au moins un pied 3 équipé du dispositif 1, pour mesurer la baropodométrie du patient 2.

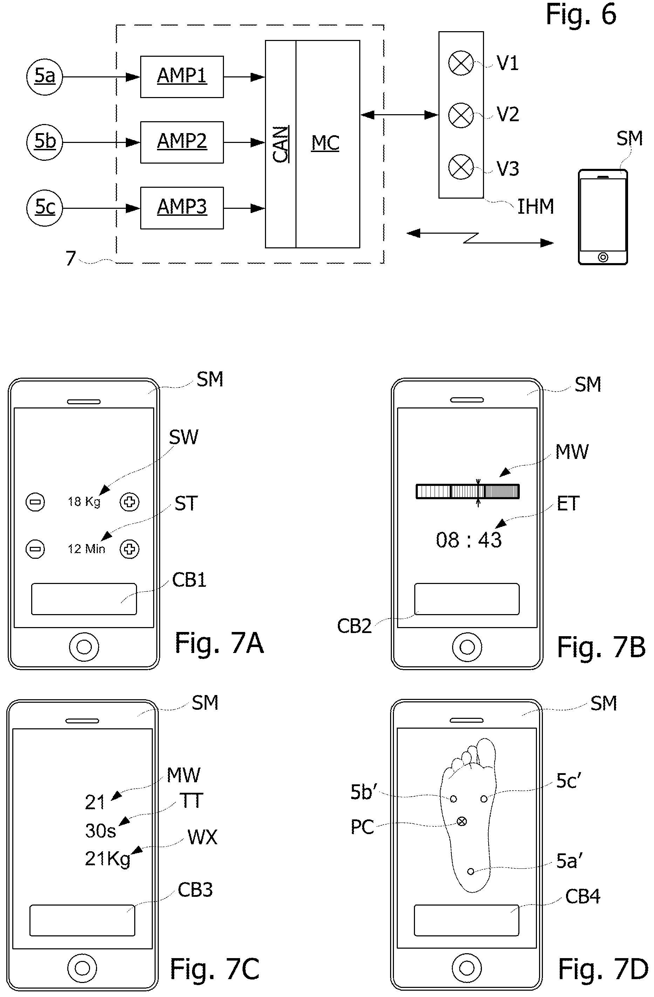

La figure 6 représente schématiquement des circuits d'un système de rééducation d'un membre inférieur, selon un mode de réalisation. Le système comprend le dispositif 1 pourvu de la carte électronique 7, et donc les capteurs 5a, 5b, 5c intégrés dans la coque 2, et une interface homme-machine IHM. La carte électronique 7 supporte une unité de traitement MC et des amplificateurs AMP1, AMP2, AMP3. Chacun des capteurs 5a, 5b, 5c est relié à l'unité de traitement MC par l'intermédiaire de liaisons conductrices et d'un amplificateur respectif parmi les amplificateurs AMP1, AMP2, AMP3. Les signaux de mesure issus des capteurs 5a-5c sont amplifiés et transmis par les amplificateurs AMP1, AMP2, AMP3 à des entrées analogiques de l'unité de traitement MC. L'unité de traitement MC convertit les signaux reçus à l'aide de convertisseurs analogiques/numériques CAN en valeurs numériques qui sont ensuite traitées par l'unité de traitement MC. L'unité de traitement MC peut être de type microprocesseur ou microcontrôleur. La carte électronique 7 peut être alimentée par une pile ou une batterie qui peut être insérée dans la fente 11. L'unité de traitement MC est reliée à l'interface homme-machine IHM par une liaison de communication filaire ou de préférence sans fil, par exemple de type Bluetooth ou BLE ("Bluetooth Low Energy").

L'unité de traitement MC peut être configurée pour calculer en temps réel ou à une certaine cadence, une valeur de force P appliquée par le pied du patient 30 sur la semelle intérieure 3 à partir des mesures fournies par les capteurs 5a-5c. La force calculée P peut être égale à la somme des forces mesurées par les capteurs 5a-5c. Comme toute les forces exercées par le pied du patient 30 sur la semelle 3 sont transmises aux capteurs 5a-5c, la valeur ainsi calculée par l'unité de traitement MC correspond à la force P effectivement exercée par le pied du patient sur la semelle 3.

Ainsi, le patient peut en permanence être informé de la qualité du pas effectué et ainsi adapter le suivant. Pour le rééducateur, l'accès en temps réel aux informations du pas effectué lui permet de guider le patient et d'ajuster ses consignes au fur et à mesure des pas.

L'unité de traitement MC peut également être configurée pour comparer les valeurs de force P calculées avec une valeur de consigne. Cette valeur de consigne peut être ajustée à l'aide d'un dispositif de réglage prévu dans l'interface IHM. En fonction des résultats de la comparaison, l'unité de traitement MC peut activer des dispositifs d'émission V1, V2, V3 de signaux lumineux et/ou sonores, prévus dans l'interface homme-machine IHM.

Par exemple, pour un certain patient, la valeur de consigne peut être ajustée à 20 kg, et des plages de 18 à 20 kg, de 16 à 18 kg, et inférieure à 16 kg peuvent être définies. Pendant la marche du patient, tout au long du déroulé du pas, le dispositif détermine en temps réel la force P exercée sur la semelle intérieure 3. Lorsque la force P calculée par l'unité de traitement MC est inférieure à 16 kg, l'interface IHM n'émet pas de signal particulier. Lorsque la force P calculée est située dans la plage entre 16 et 18 kg, un dispositif d'émission V1 tel qu'une LED verte est activé, indiquant que la force P exercée est satisfaisante. Lorsque la force P est située dans la plage entre 18 et 20 kg, un dispositif d'émission V2 tel qu'une LED rouge est activé, indiquant que la force P exercée est légèrement trop élevée. Lorsque la force P calculée est supérieure à 20 kg, un dispositif d'émission tel qu'un avertisseur sonore V3 est déclenché, le dispositif d'émission V2 étant actif, pour indiquer que la force P exercée est trop élevée et peut être néfaste pour la guérison.

Grâce à ces dispositions, le patient 30 peut apprendre à adapter sa démarche pour limiter la force exercée par sa jambe en rééducation durant la marche, afin de favoriser sa guérison. L'interface IHM fournit ainsi au patient une stimulation sensorielle permettant une rétroaction neuromusculaire stimulant la neuro-plasticité et la reprogrammation sensorimotrice des patients.

Selon un mode de réalisation, l'unité de traitement MC est configurée pour calculer en temps réel la position d'un centre d'appui du pied, correspondant au barycentre des forces mesurées par les capteurs 5a, 5b, 5c, et transmettre cette position à l'interface IHM.

Selon un mode de réalisation, l'unité de traitement MC ou la carte électronique 7 est configurée pour pouvoir communiquer avec un terminal mobile tel qu'un "smartphone". Les figures 7A, 7B, 7C et 7D représentent un tel terminal mobile SM exécutant une application dédiée. L'écran du terminal SM est ainsi utilisé pour émettre des signaux visuels et/ou sonores dans le cadre d'une interface homme-machine.

La figure 7A montre une image affichée à l'écran du terminal SM présentant des boutons de commande permettant le réglage d'une valeur de force de consigne SW et d'une durée d'exercice ST, et un bouton de commande CB1 permettant de valider le réglage. La validation du réglage à l'aide du bouton CB1 déclenche l'affichage de l'image présentée sur la figure 7B. L'image montrée sur la figure 7B présente une barre MW indiquant différentes plages de valeurs de force P calculée et transmise par l'unité de traitement MC et la durée ET de l'exercice en cours. Un bouton d'arrêt CB2 affiché sur l'image de la figure 7B permet d'arrêter l'exercice en cours. La figure 7C montre un compte-rendu de série d'exercices. Ce compte-rendu peut comprendre par exemple un nombre d'exercices MW, le temps total écoulé TT durant les exercices et la force maximale mesurée. La figure 7D montre une image affichée à l'écran du terminal SM présentant le contour d'un pied humain, et en relation avec le contour du pied, les positions respectives 5a', 5b', 5c' des capteurs 5a, 5b, 5c, et la position du centre de pression PC calculée par l'unité de traitement MC en fonction des mesures fournies par les capteurs 5a, 5b, 5c. La sortie de cette présentation de la position du centre de pression PC est effectuée à l'aide d'un bouton de commande CB4.

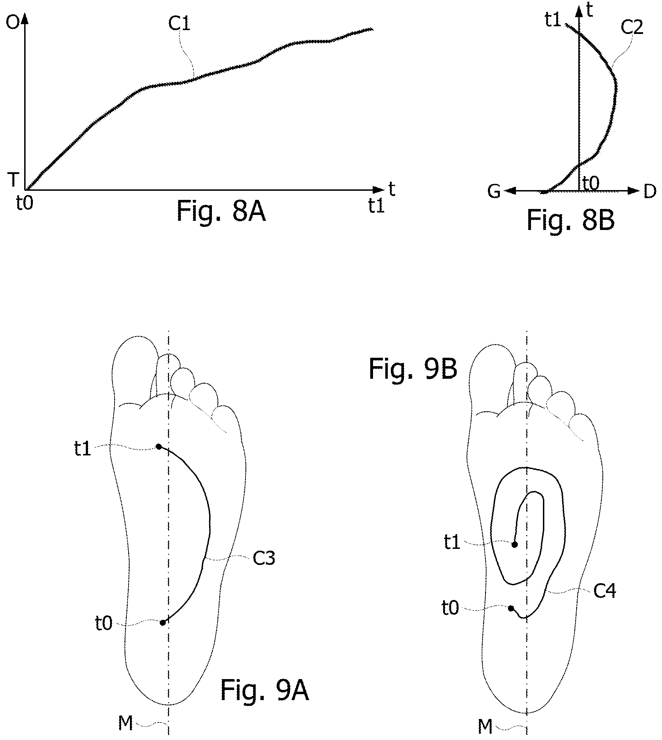

Selon un mode de réalisation, l'unité de traitement MC ou la carte électronique 7 est configurée pour déterminer les variations en fonction du temps de la position du centre de pression PC du pied en fonction des mesures fournies par les capteurs 5a, 5b, 5c. En outre, l'unité de traitement MC peut être configurée pour exploiter les variations de la position du centre de pression PC en affichant des courbes ou des chronogrammes. Des exemples de chronogrammes susceptibles d'être affichés par l'unité de traitement MC sont présentés dans les figures 8A, 8B et 9A, 9B.

Ainsi, les figures 8A, 8B représentent des chronogrammes de variation de la position du centre de pression du pied pendant le déroulé du pas, respectivement vers O ou l'arrière T du pied, et vers la gauche G ou la droite D du pied. Sur la figure 8A, le chronogramme C1 montre que le centre de pression PC se déplace progressivement du talon T à l'instant t0 jusqu'aux orteils O à l'instant t1. Sur la figure 8B, le chronogramme C2 montre que le centre de pression PC se déplace de la gauche G d'une ligne médiane du pied vers la droite D de cette ligne médiane, pour finalement revenir à gauche de la ligne médiane.

Les figures 9A, 9B représentent des exemples de courbes de variation de la position du centre de pression du pied en statique, affichées en surimpression sur une image du contour du pied, entre des instants t0 et t1. Dans l'exemple de la figure 9A, le centre de pression se trouve à l'instant t0 proche du talon et légèrement à gauche de la ligne médiane M du pied. Ensuite, le centre de pression suit une courbe C3 vers une position extrême à droite de la ligne médiane M et vers l'avant du pied, pour finir à l'instant t1 en une position proche des orteils et légèrement à gauche de la ligne médiane M.

Dans l'exemple de la figure 9B, le centre de pression suit une courbe C4 en forme de spirale convergente. La courbe C4 part, à l'instant t0, d'une position située vers le talon et à droite de la ligne médiane M, pour aller vers une position finale à l'instant t1 située vers le centre du pied, légèrement à droite de la ligne médiane. La courbe C4 s'étend largement vers la droite et vers la gauche du pied.

Il apparaîtra clairement à l'homme de l'art que la présente invention est susceptible de diverses variantes de réalisation et diverses applications. En particulier, l'invention n'est pas limitée à un dispositif comportant une ou plusieurs vis 8a, 8b. En effet, ces vis peuvent être omises, sachant qu'elles assurent seulement le maintien de la semelle intérieure 3 dans la coque 2, lorsque cette dernière est renversée vers le bas. En outre, lorsque le dispositif de mesure 1 est utilisé, avec le pied d'un patient dans la coque 2, la semelle intérieure 3 est maintenue dans la coque par le pied. Par ailleurs d'autres moyens peuvent remplacer les vis 8a, 8b, comme des crochets agencés sur la coque 2 et coopérant avec le bord de la semelle intérieure 3.

Il n'est pas non plus nécessaire de prévoir une coque avec un rebord entourant la semelle intérieure 3. Seul un support pour les capteurs 5a-5c, la carte électronique 7 et la semelle 3 peut suffire, notamment si l'on prévoit des vis telles que les vis 8a, 8b et leur agencement pour solidariser la semelle au support sans transmettre les forces appliquées sur la semelle.

Par ailleurs, tout ou partie des traitements décrits précédemment comme étant effectués par l'unité de traitement MC peuvent être réalisés par un équipement externe tel que l'interface IHM ou le terminal SM. Ainsi, l'unité de traitement MC peut simplement être configurée pour calculer en temps réel une valeur de force appliquée par le pied du patient à partir des signaux de mesure fournies par les capteurs 5a-5c, et transmettre en temps réel la valeur de force calculée à l'équipement externe. L'unité de traitement MC peut plus simplement être configurée pour collecter et numériser les signaux de mesure issus des capteurs 5a, 5b, 5c, et les transmettre à l'équipement externe. L'unité de traitement MC peut encore plus simplement être configurée pour mémoriser les signaux issus des capteurs et numérisés, par exemple dans une carte mémoire amovible. La carte mémoire peut ensuite être lue par l'équipement externe, lequel peut être configuré pour établir et afficher un compte-rendu d'exercice à partir des données lues dans la carte mémoire.

En outre, le nombre de capteurs 5a-5c installés dans la coque 2 peut être différent de trois, par exemple supérieur à trois, notamment si une plus grande précision dans la détermination de la position du centre de pression PC est requise.

## Revendications

1. Dispositif de mesure d'une force d'appui exercée par le pied d'un patient, le dispositif comprenant :
un support (2) ;
une semelle intérieure (3) prévue pour supporter le pied du patient, maintenue sur le support, le support et la semelle étant agencés pour que, lorsque le pied du patient est en appui sur la semelle intérieure, les forces exercées par le pied du patient sur la semelle intérieure soient transmises au support en un ensemble fini de zones de contact séparées ;
des capteurs (5a, 5b, 5c) interposés entre le support et la semelle intérieure respectivement aux zones de contact, et configurés chacun pour fournir un signal représentatif d'une force exercée sur la zone de contact correspondante ; et
une unité de traitement (MC) connectée aux capteurs et configurée pour acquérir les signaux de force fournis par les capteurs.

2. Dispositif selon la revendication 1, comprenant un organe de retenue (8a, 8b) agencé entre la semelle intérieure (3) et le support (2) en une zone de retenue distincte des zones de contact, pour solidariser la semelle intérieure au support sans transmettre les forces exercées sur la semelle au support.

3. Dispositif selon la revendication 2, dans lequel l'organe de retenue (8a, 8b) est configuré pour s'engager partiellement dans un orifice au travers du support (2) et venir se fixer dans la semelle intérieure (3), de sorte que l'organe de retenue soit retenu par le support vers le haut avec un jeu non nul.

4. Dispositif selon l'une des revendications 1 à 3, dans lequel le support (2) comprend une semelle extérieure (10) de forme bombée le long d'un axe longitudinal du support (2).

5. Dispositif selon l'une des revendications 1 à 4, dans lequel les capteurs (5a, 5b, 5c) comprennent trois capteurs de force incluant un capteur (5a) disposé sous une partie arrière de la semelle intérieure (3), et deux capteurs (5b, 5c) disposés sous une partie avant de la semelle intérieure.

6. Dispositif selon l'une des revendications 1 à 5, dans lequel le support présente la forme d'une coque (2) avec un rebord (2a) entourant la semelle (3).

7. Dispositif selon l'une des revendications 1 à 6, dans lequel :
l'unité de traitement (MC) est configurée pour calculer en temps réel une valeur de force (P) appliquée par le pied du patient sur la semelle intérieure (3) à partir des signaux fournis par les capteurs (5a, 5b, 5c), et transmettre la valeur de force vers un équipement externe (IHM, SM), et/ou
l'unité de traitement (MC) est configurée pour transmettre en temps réel des mesures représentatives des signaux fournis par les capteurs à l'équipement externe (IHM, SM), l'équipement externe étant configuré pour calculer en temps réel une valeur de force (P) appliquée par le pied du patient sur la semelle intérieure (3) à partir des mesures fournies par l'unité de traitement.

8. Système de rééducation d'un membre inférieur d'un patient, le système comprenant :
un dispositif de mesure (1) selon l'une des revendications 1 à 7, et
une interface homme-machine (IHM, SM) en communication avec l'unité de traitement (MC) du dispositif de mesure et configurée pour émettre en temps réel un signal représentatif d'une valeur de force (P) calculée à partir des signaux de force fournis par les capteurs (5a, 5b, 5c).

9. Système selon la revendication 8, dans lequel l'interface homme-machine comprend un terminal mobile (SM) en communication avec l'unité de traitement (MC) et muni d'une application dédiée configurée pour traiter des signaux transmis par l'unité de traitement.

10. Système selon la revendication 8 ou 9, dans lequel l'interface homme-machine (IHM) est configurée pour émettre en temps réel :
un premier signal lumineux et/ou sonore lorsque la valeur de force (P) est comprise dans une première plage de valeurs de forces, et
un second signal lumineux et/ou sonore lorsque la valeur de force (P) est comprise dans une seconde plage de valeurs de forces, supérieure à la première plage de valeurs de force, et inférieure à une valeur de consigne.

11. Système selon l'une des revendications 8 à 10, dans lequel l'unité de traitement (MC) ou l'interface homme-machine (IHM, SM) est configurée pour calculer en temps réel la position d'un centre de pression (PC) situé au barycentre des forces exercées sur les zones de contact et mesurées par les capteurs (5a, 5b, 5c).

12. Système selon la revendication 11, dans lequel l'interface homme-machine (IHM, SM) est configurée pour afficher la position du centre de pression (PC) en relation avec le contour d'un pied humain, et/ou des variations au cours du temps de la position du centre de pression sous forme de chronogrammes et/ou en relation avec le contour d'un pied humain.

## Patentansprüche

1. Vorrichtung zum Messen einer Auflagekraft, die von dem Fuß eines Patienten ausgeübt wird, wobei die Vorrichtung umfasst:
eine Stütze (2);
eine Innensohle (3), die dafür vorgesehen ist, den Fuß des Patienten, der an die Stütze gehalten wird, abzustützen, wobei die Stütze und die Sohle so eingerichtet sind, dass wenn der Fuß des Patienten auf der Innensohle aufliegt, die Kräfte, die von dem Fuß des Patienten auf die Innensohle ausgeübt werden, in einer endlichen Menge von getrennten Kontaktbereichen auf die Stütze übertragen werden;
Sensoren (5a, 5b, 5c), die jeweils an den Kontaktbereichen zwischen der Stütze und der Innensohle eingefügt und jeweils so ausgelegt sind, dass sie ein Signal liefern, das für eine auf den entsprechenden Kontaktbereich ausgeübte Kraft repräsentativ ist; und
eine Verarbeitungseinheit (MC), die mit den Sensoren verbunden und so ausgelegt ist, dass sie die von den Sensoren gelieferten Kraftsignale erfasst.

2. Vorrichtung nach Anspruch 1, die ein Halteelement (8a, 8b) umfasst, das zwischen der Innensohle (3) und der Stütze (2) in einem von den Kontaktbereichen getrennten Haltebereich angeordnet ist, um die Innensohle mit der Stütze zu verbinden, ohne die auf die Sohle ausgeübten Kräfte auf die Stütze zu übertragen.

3. Vorrichtung nach Anspruch 2, wobei das Halteelement (8a, 8b) so ausgelegt ist, dass es teilweise in eine Öffnung durch die Stütze (2) einrückt und sich in der Innensohle (3) befestigt, sodass das Halteelement von der Stütze nach oben mit einem Spiel von ungleich null gehalten wird.

4. Vorrichtung nach einem der Ansprüche 1 bis 3, wobei die Stütze (2) eine Außensohle (10) von gewölbter Form entlang einer Längsachse der Stütze (2) umfasst.

5. Vorrichtung nach einem der Ansprüche 1 bis 4, wobei die Sensoren (5a, 5b, 5c) drei Kraftsensoren umfassen, die einen Sensor (5a), der unter einem hinteren Teil der Innensohle (3) angeordnet ist, und zwei Sensoren (5b, 5c) einschließen, die unter einem vorderen Teil der Innensohle angeordnet sind.

6. Vorrichtung nach einem der Ansprüche 1 bis 5, wobei die Stütze die Form einer Schale (2) mit einem die Sohle (3) umgebenden Rand (2a) aufweist.

7. Vorrichtung nach einem der Ansprüche 1 bis 6, wobei:
die Verarbeitungseinheit (MC) so ausgelegt ist, dass sie in Echtzeit einen von dem Fuß des Patienten auf die Innensohle (3) aufgebrachten Kraftwert (P) auf Grundlage der von den Sensoren (5a, 5b, 5c) gelieferten Signale berechnet und den Kraftwert an eine externe Ausrüstung (MMI, SM) überträgt, und/oder
die Verarbeitungseinheit (MC) so ausgelegt ist, dass sie in Echtzeit Messungen, die für die von den Sensoren gelieferten Signale repräsentativ sind, an die externe Ausrüstung (MMI, SM) überträgt, wobei die externe Ausrüstung so ausgelegt ist, dass sie in Echtzeit einen Kraftwert (P), der von dem Fuß des Patienten auf die Innensohle (3) aufgebracht wird, auf Grundlage der von der Verarbeitungseinheit gelieferten Messungen berechnet.

8. Rehasystem für eine untere Extremität eines Patienten, wobei das System umfasst:
eine Messvorrichtung (1) nach einem der Ansprüche 1 bis 7 und eine Mensch-Maschine-Schnittstelle (HMI, SM), die mit der Verarbeitungseinheit (MC) der Messvorrichtung in Kommunikation steht und so ausgelegt ist, dass sie in Echtzeit ein Signal ausgibt, das für einen auf Grundlage der von den Sensoren (5a, 5b, 5c) gelieferten Kraftsignale berechneten Kraftwert (P) repräsentativ ist.

9. System nach Anspruch 8, wobei die Mensch-Maschine-Schnittstelle ein mobiles Endgerät (SM) umfasst, das mit der Verarbeitungseinheit (MC) in Kommunikation steht und mit einer dedizierten Anwendung ausgestattet ist, die so ausgelegt ist, dass sie von der Verarbeitungseinheit übertragene Signale verarbeitet.

10. System nach Anspruch 8 oder 9, wobei die Mensch-Maschine-Schnittstelle (HMI) so ausgelegt ist, dass sie in Echtzeit ausgibt:
ein erstes Leucht- und/oder Tonsignal, wenn der Kraftwert (P) in einem ersten Kraftwertbereich liegt, und
ein zweites Leucht- und/oder Tonsignal, wenn der Kraftwert (P) in einem zweiten Kraftwertbereich liegt, der größer ist als der erste Kraftwertbereich, und kleiner ist als ein Sollwert.

11. System nach einem der Ansprüche 8 bis 10, wobei die Verarbeitungseinheit (MC) oder die Mensch-Maschine-Schnittstelle (HMI, SM) so ausgelegt ist, dass sie in Echtzeit die Position eines Druckzentrums (PC) berechnet, das sich im Schwerpunkt der auf die Kontaktbereiche ausgeübten und von den Sensoren (5a, 5b, 5c) gemessenen Kräfte befindet.

12. System nach Anspruch 11, wobei die Mensch-Maschine-Schnittstelle (MMI, SM) so ausgelegt ist, dass sie die Position des Druckzentrums (PC) in Bezug auf die Kontur eines menschlichen Fußes und/oder Änderungen der Position des Druckzentrums über die Zeit hinweg in Form von Chronogrammen und/oder in Bezug auf die Kontur eines menschlichen Fußes anzeigt.

## Claims

1. A device for measuring a support force exerted by a patient's foot, the device comprising:
a support (2);
an insole (3) provided to support the patient's foot, held on the support, the support and the insole being arranged so that, when the patient's foot bears on the insole, the forces exerted by the patient's foot on the insole are transmitted to the support at a finite set of discrete contact areas;
sensors (5a, 5b, 5c) interposed between the support and the insole respectively at the contact areas, and each configured to provide a signal representative of a force exerted on the corresponding contact area; and
a processing unit (MC) connected to the sensors and configured to acquire the force signals provided by the sensors.

2. The device according to claim 1, comprising a retaining member (8a, 8b) arranged between the insole (3) and the support (2) in a retention area separate from the contact areas, for securing the insole to the support without transmitting the forces exerted on the insole to the support.

3. The device according to claim 2, wherein the retaining member (8a, 8b) is configured to partially engage in an orifice through the support (2) and to be fastened in the insole (3), so that the retaining member is retained upwardly by the support with a non-zero clearance.

4. The device according to any of claims 1 to 3, wherein the support (2) comprises an outsole (10) with a convex shape along a longitudinal axis of the support (2).

5. The device according to any of claims 1 to 4, wherein the sensors (5a, 5b, 5c) comprise three force sensors including one sensor (5a) disposed under a rear part of the insole (3), and two sensors (5b, 5c) disposed under a front part of the insole.

6. The device according to any of claims 1 to 5, wherein the support has the shape of a shell (2) with a rim (2a) surrounding the insole (3).

7. The device according to any of claims 1 to 6, wherein:
the processing unit (MC) is configured to calculate in real time a force value (P) applied by the patient's foot on the insole (3) from the signals provided by the sensors (5a, 5b, 5c), and transmit the force value to an external equipment (HMI, SM), and/or
the processing unit (MC) is configured to transmit in real time measurements representative of the signals provided by the sensors to the external equipment (HMI, SM), the external equipment being configured to calculate in real time a force value (P) applied by the patient's foot on the insole (3) from the measurements provided by the processing unit.

8. A system for rehabilitating a lower limb of a patient, the system comprising:
a measuring device (1) according to any of claims 1 to 7, and
a human-machine interface (HMI, SM) communicating with the processing unit (MC) of the measuring device and configured to emit in real time a signal representative of a force value (P) calculated from the force signals provided by the sensors (5a, 5b, 5c).

9. The system according to claim 8, wherein the human-machine interface comprises a mobile terminal (SM) communicating with the processing unit (MC) and provided with a dedicated application configured to process signals transmitted by the processing unit.

10. The system according to claim 8 or 9, wherein the human-machine interface (HMI) is configured to emit in real time:
a first light and/or sound signal when the force value (P) is within a first range of force values, and
a second light and/or sound signal when the force value (P) is within a second range of force values, greater than the first range of force values, and less than a setpoint value.

11. The system according to any of claims 8 to 10, wherein the processing unit (MC) or the human-machine interface (HMI, SM) is configured to calculate in real time the position of a center of pressure (PC) located at the barycenter of the forces exerted on the contact areas and measured by the sensors (5a, 5b, 5c).

12. The system according to claim 11, wherein the human-machine interface (HMI, SM) is configured to display the position of the center of pressure (PC) in relation to the contour of a human foot, and/or variations over time in the position of the center of pressure in the form of chronograms and/or in relation to the contour of a human foot.
